# EUROPEAN PATENT APPLICATION

(11) **EP 4 785 796 A2**
(43) Date of publication of application: **05.08.2026**
(21) Application number: 26186055.5
(22) Date of filing: 08.06.2021
(51) Int. Cl.: A01P 7/00

(54) **SOLID STATE FORMS OF CYANTRANILIPROLE**

(30) Priority: 08.06.2020 US 202063035865 P
(62) Divisional of application: 21821439.3
(71) Applicant: ADAMA MAKHTESHIM LTD., 8410001 Beer Sheva (IL)
(72) Inventor: SUEZ, Gal, 8619006 Dimona (IL); FORCKOSH, Hagit, 8479154 Beer Sheva (IL); GOLUB, Yanai, 8496501 Omer (IL); LIE, Jie, Nanjing, Jiangsu, 210046 (CN); CHEN, Bob, Shanghai, 201199 (CN); PARNES, Regev, 8531500 Kibbutz Mishmar Hanegev (IL)
(74) Representative: Modiano, Gabriella Diana

(57) **Abstract**

Solid state forms of Cyantraniliprole, processes for preparation and agrochemical compositions thereof.

## Description

### Technical Field

The present disclosure relates to solid state forms of Cyantraniliprole, processes for preparation thereof and agrochemical compositions thereof.

### Background of the Disclosure

The control of invertebrate pests is extremely important in achieving high crop efficiency. Damage by invertebrate pests to growing and stored agronomic crops can cause significant reduction in productivity and thereby result in increased costs to the consumer. Certain anthranilamides, their N-oxides, salts and compositions suitable for agronomic and non-agronomic uses, and a method of their use for controlling invertebrate pests in both agronomic and non-agronomic environments. The control of invertebrate pests in forestry, greenhouse crops, ornamentals, nursery crops, stored food and fiber products, livestock, household, and public and animal health is also important.

Cyantraniliprole is an anthranilamide insecticide of the ryanoid class. It is a systemic diamide insecticide used specifically to control lepidopteran pests in agriculture. It is a promising insecticide due to its unique mode of action in activating muscle ryanodine receptors, causing contraction and paralysis of some pests. It has already been registered for use against lepidopterous pests and aphids on several fruits and vegetables including apples, peaches, tomatoes, watermelons, and cucumbers.

Cyantraniliprole has the chemical name, 3-Bromo-1-(3-chloro-2-pyridinyl)-N-[4-cyano-2-methyl-6-(methylcarbamoyl)phenyl]-1H-pyrazole-5-carboxamide.

Cyantraniliprole has the following chemical structure:

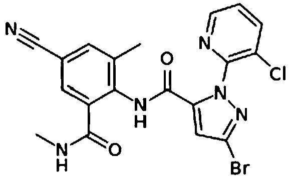

Cyantraniliprole, an N-oxide or a salt, and process thereof, was first disclosed by E.I. du Pont Company in the International publication, WO 2004/067528. Further processes for its preparation were disclosed in WO 2006/062978 and WO 2010/056720.

Furthermore, methods were disclosed in the WO2010056720 publication for the preparation of a non-hydratable polymorph A of Cyantraniliprole from a hydratable polymorph B of Cyantraniliprole.

Polymorphism, the occurrence of different crystal forms, is a property of some molecules and molecular complexes. A single compound, like Cyantraniliprole, may give rise to a variety of polymorphs having distinct crystal structures and physical properties like melting point, thermal behaviours (e.g. measured by thermogravimetric analysis - "TGA", or differential scanning calorimetry - "DSC"), X-ray powder diffraction (XRPD) pattern, infrared absorption fingerprint, Raman absorption fingerprint, and solid state (¹³C-) NMR spectrum. One or more of these techniques may be used to distinguish different polymorphic forms of a compound.

Different salts and solid state forms (including solvated forms) of an active ingredient may possess different properties. Such variations in the properties of different salts and solid state forms and solvates may provide a basis for improving formulation, for example, by facilitating better processing or handling characteristics, improving the dissolution profile, or improving stability (polymorph as well as chemical stability) and shelf-life. These variations in the properties of different salts and solid state forms may also provide improvements to the final formulation or recipe, for instance, if they serve to improve dissolution. Different salts and solid state forms and solvates of an active ingredient may also give rise to a variety of polymorphs or crystalline forms, which may in turn provide additional opportunities to use variations in the properties and characteristics of a solid active ingredient for providing an improved product.

Discovering new salts, solid state forms and solvates of an agrochemical product can provide materials having desirable processing properties, such as ease of handling, ease of processing, storage stability, and ease of purification or as desirable intermediate crystal forms that facilitate conversion to other salts or polymorphic forms. New salts, polymorphic forms and solvates of an active ingredient can also provide an opportunity to improve the performance characteristics of an agrochemical product (dissolution profile, permeability, etc.). It expands the range of materials that an expert in the field can avail for formulation optimization, for example by providing a product with different properties, e.g., a different crystal habit, higher crystallinity or polymorphic stability which may offer better processing or handling characteristics, improved dissolution profile, or improved shelf-life.

For at least these reasons, there is a need for some solid state forms (including solvated forms or salts) of Cyantraniliprole.

### Summary

The present disclosure relates to solid state form of Cyantraniliprole, processes for preparation thereof, and agrochemical compositions including this solid state form.

The present disclosure also provides use of the solid state form of Cyantraniliprole for preparing other solid state forms of Cyantraniliprole, Cyantraniliprole N-oxide and solid state forms of a Cyantraniliprole salt.

In an embodiment, the present disclosure provides use of Cyantraniliprole Form F for preparing Form D of Cyantraniliprole.

The present disclosure also provides solid state form of Cyantraniliprole for uses in the preparation of other solid state forms of Cyantraniliprole, Cyantraniliprole N-oxide and solid state forms of a Cyantraniliprole salts.

In one embodiment, the present disclosure encompasses the described solid state forms of Cyantraniliprole for use in the preparation of agrochemical compositions and/or formulations, optionally for agronomic and non-agronomic uses, and a method of their use for controlling invertebrate pests in both agronomic and non-agronomic environments.

In another embodiment, the present disclosure encompasses uses of the described solid state forms of Cyantraniliprole for the preparation of agrochemical compositions and/or formulations.

The present disclosure further provides agrochemical compositions including one or more solid state forms of Cyantraniliprole according to the present disclosure.

In yet another embodiment, the present disclosure encompasses agrochemical formulations including one or more of the described solid state forms of Cyantraniliprole and at least one additional component selected from the group consisting of a surfactant, a solid diluent and liquid diluent and optionally an effective amount of at least one additional biologically active compound or agent.

The present disclosure encompasses processes to prepare said agrochemical formulations of Cyantraniliprole including one or more of the described solid state forms and at least one additional component selected from the group consisting of a surfactant, a solid diluent and liquid diluent and optionally an effective amount of at least one additional biologically active compound or agent.

The solid state forms defined herein as well as the agrochemical compositions or formulations of the solid state forms of Cyantraniliprole can be used as systemic diamide insecticide for controlling invertebrate pests in both agronomic and non-agronomic environments.

The present disclosure also provides methods for controlling invertebrate pests comprising applying solid state forms of Cyantraniliprole by activating muscle ryanodine receptors, causing contraction and paralysis of some pests.

The present disclosure also provides uses of the solid state forms of Cyantraniliprole of the present disclosure, or at least one of the above agrochemical compositions or formulations for controlling invertebrate pests in both agronomic and non-agronomic environments.

### Brief Description of the Figures

Figure 1 shows an X-ray powder diffractogram (XRPD) of Form C of Cyantraniliprole.
Figure 2 shows an X-ray powder diffractogram (XRPD) of Form D of Cyantraniliprole.
Figure 3 shows an X-ray powder diffractogram (XRPD) of Form F of Cyantraniliprole.

### Detailed Description

The present disclosure relates to solid state forms of Cyantraniliprole, processes for preparation thereof and agrochemical compositions including the solid state form. The disclosure also relates to the conversion of the described solid state form of Cyantraniliprole to other solid state forms of Cyantraniliprole, Cyantraniliprole N-oxide, Cyantraniliprole salts and their solid state forms thereof.

The solid state forms of Cyantraniliprole according to the present disclosure may have advantageous properties selected from at least one of: chemical or polymorphic purity, flowability, solubility, dissolution rate, morphology or crystal habit, stability - such as chemical stability as well as thermal and mechanical stability with respect to polymorphic conversion, stability towards dehydration and/or storage stability, a lower degree of hygroscopicity, low content of residual solvents and advantageous processing and handling characteristics such as compressibility, or bulk density.

A crystal form may be referred to herein as being characterized by graphical data as depicted in a Figure. Such data include, for example, powder X-ray diffractograms and solid state NMR spectra. As is well-known in the art, the graphical data potentially provides additional technical information to further define the respective solid state form which can not necessarily be described by reference to numerical values or peak positions alone. In any event, the skilled person will understand that such graphical representations of data may be subject to small variations, e.g., in peak relative intensities and peak positions due to factors such as variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of Cyantraniliprole referred to herein as being characterized by graphical data "as depicted in" a Figure will thus be understood to include any crystal form of Cyantraniliprole, characterized with the graphical data having such small variations, as are well known to the skilled person, in comparison with the Figure.

A pure solid state form (or polymorph) may be referred to herein as polymorphically pure or as substantially free of any other solid state (or polymorphic) forms. As used herein in this context, the expression "substantially free of any other forms" will be understood to mean that the solid state form contains about 20% or less, about 10% or less, about 5% or less, about 2% or less, about 1% or less, or 0% of any other forms of the subject compound as measured, for example, by XRPD. Thus, the solid state form of Cyantraniliprole described herein as substantially free of any other solid state forms would be understood to contain greater than about 80% (w/w), greater than about 90% (w/w), greater than about 95% (w/w), greater than about 98% (w/w), greater than about 99% (w/w), or 100% of the subject solid state form of Cyantraniliprole. Accordingly, in some embodiments of the disclosure, the described solid state form of Cyantraniliprole may contain from about 1% to about 20% (w/w), from about 5% to about 20% (w/w), or from about 5% to about 10% (w/w) of one or more other solid state forms of the same Cyantraniliprole.

In an aspect the present disclosure contemplates that a certain solid state form of Cyantraniliprole can exist in the presence of any other of the solid state forms or mixture thereof. Accordingly, in one embodiment, the present disclosure provides form D, for example, wherein form D is present in a solid form that includes less than 95%, less than 90%, less than 80%, less than 70%, less than 60%, less than 50%, less than 40%, less than 30%, less than 20%, less than 10%, less than 5%, less than 3% or less than 1% by weight of any other physical forms of Cyantraniliprole.

The solid state forms of Cyantraniliprole as characterized herein may be present also in traces in an agrochemical compositions or formulations for controlling invertebrate pests in both agronomic and non-agronomic environments.

As used herein, unless stated otherwise, XRPD peaks reported herein are optionally measured using CuK_{α}, radiation, λ = 1.54187 Å.

As used herein, the term "isolated" in reference to solid state forms of Cyantraniliprole of the present disclosure corresponds to solid state form of Cyantraniliprole that is physically separated from the reaction mixture in which it is formed.

A thing, e.g., a reaction mixture, may be characterized herein as being at, or allowed to come to "room temperature", often abbreviated "RT." This means that the temperature of the thing is close to, or the same as, that of the space, e.g., the room or fume hood, in which the thing is located. Typically, room temperature is from about 20°C to about 30°C, about 22°C to about 27°C, or about 25°C.

A process or step may be referred to herein as being carried out "overnight." This refers to a time interval, e.g., for the process or step, that spans the time during the night, when that process or step may not be actively observed. This time interval is from about 8 to about 20 hours, about 10 to about 18 hours, or about 16 hours.

The amount of solvent employed in a chemical process, e.g., a reaction or crystallization, may be referred to herein as a number of "volumes" or "vol" or "V." For example, a material may be referred to as being suspended in 10 volumes (or 10 vol or 10V) of a solvent. In this context, this expression would be understood to mean milliliters of the solvent per gram of the material being suspended, such that suspending a 5 grams of a material in 10 volumes of a solvent means that the solvent is used in an amount of 10 milliliters of the solvent per gram of the material that is being suspended or, in this example, 50 mL of the solvent. In another context, the term "v/v" may be used to indicate the number of volumes of a solvent that are added to a liquid mixture based on the volume of that mixture. For example, adding methyl tert-butyl ether (MTBE) (1.5 v/v) to a 100 ml reaction mixture would indicate that 150 mL of MTBE was added.

As used herein, the term "reduced pressure" refers to a pressure of about 10 mbar to about 50 mbar.

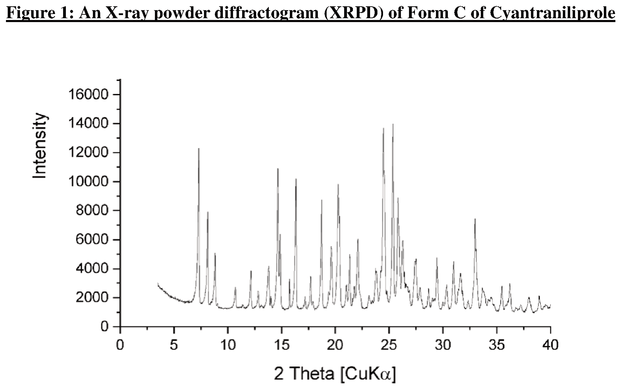

The present disclosure includes a solid state form of Cyantraniliprole designated as Form C. The Form C of Cyantraniliprole can be characterized by data selected from one or more of the following: an XRPD pattern having peaks at 7.3, 8.2, 8.9, 14.7 and 20.3 degrees 2-theta ± 0.2 degrees 2-theta; an XRPD pattern as depicted in Figure 1; and combinations of these data.

Crystalline Form C of Cyantraniliprole may be further characterized by the XRPD pattern having peaks at 7.3, 8.2, 8.9, 14.7 and 20.3 degrees 2-theta ± 0.2 degrees 2-theta, and also having one, two or three additional peaks selected from 16.3, 18.7, 24.5 and 25.4 degrees 2- theta ± 0.2 degrees 2-theta.

Crystalline Form C of Cyantraniliprole may be characterized by any combination of at least four peaks in an XRPD pattern as depicted in Figure 1.

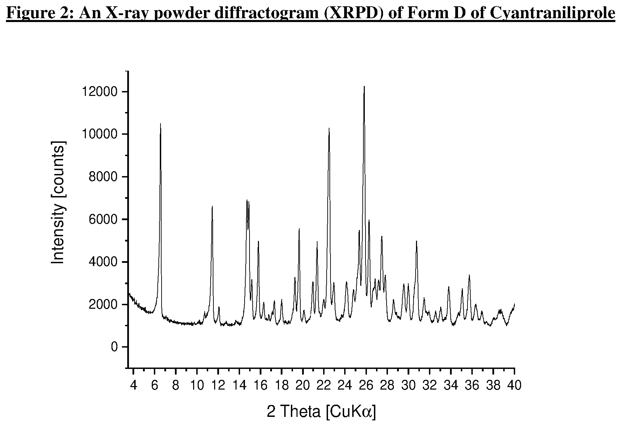

The present disclosure also includes a solid state form of Cyantraniliprole designated as Form D. The Form D of Cyantraniliprole can be characterized by data selected from one or more of the following: an XRPD pattern having peaks at 6.6, 11.4, 15.7 and 22.4 degrees 2-theta ± 0.2 degrees 2-theta; an XRPD pattern as depicted in Figure 2; and combinations of these data.

Crystalline Form D of Cyantraniliprole may be further characterized by the XRPD pattern having peaks at 6.6, 11.4, 15.7 and 22.4 degrees 2-theta ± 0.2 degrees 2-theta, and also having one, two or three additional peaks selected from 14.7, 19.6, 21.4 and 25.7 degrees 2- theta ± 0.2 degrees 2-theta.

Crystalline Form D of Cyantraniliprole may be characterized by any combination of at least four peaks in an XRPD pattern as depicted in Figure 2.

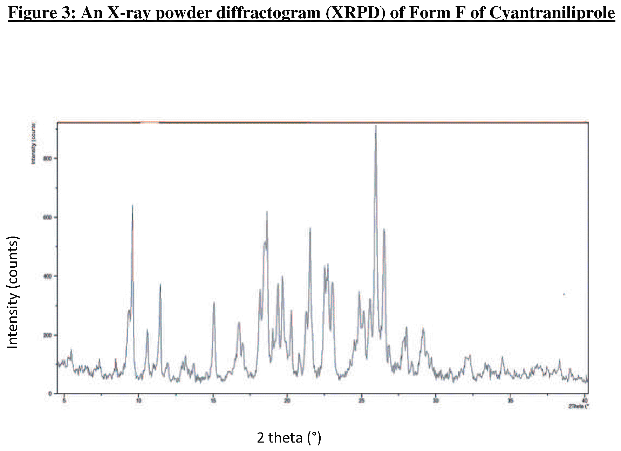

The present disclosure further includes a solid state form of Cyantraniliprole designated as Form F. The Form F of Cyantraniliprole can be characterized by data selected from one or more of the following: an XRPD pattern having peaks at 9.6, 11.3, 18.6, 21.5 and 25.9 degrees 2-theta ± 0.2 degrees 2-theta; an XRPD pattern as depicted in Figure 3; and combinations of these data.

Crystalline Form F of Cyantraniliprole may be further characterized by the XRPD pattern having peaks at 9.6, 11.3, 18.6, 21.5 and 25.9 degrees 2-theta ± 0.2 degrees 2-theta, and also having one, two or three additional peaks selected from 15.1, 16.7, 19.7, 20.3 and 26.5 degrees 2- theta ± 0.2 degrees 2-theta.

Crystalline Form F of Cyantraniliprole may be characterized by any combination of at least four peaks in an XRPD pattern as depicted in Figure 3.

The present disclosure also provides the solid state forms of Cyantraniliprole of the present disclosure for use in the preparation of other solid state forms of Cyantraniliprole, Cyantraniliprole N-oxide, Cyantraniliprole salts and solid state forms thereof.

The present disclosure further encompasses processes for preparing Cyantraniliprole solid state forms. The process includes starting from an intermediate, 2-[3-Bromo-1-(3-chloro-2-pyridinyl)-1H-pyrazol-5-yl]-6-cyano-8-methyl-4H-3,1-benzoxazin-4-one, and converting it to a Cyantraniliprole solid state form. The conversion can be done, for example, by a process including subjecting a solution of 2-[3-Bromo-1-(3-chloro-2-pyridinyl)-1H-pyrazol-5-yl]-6-cyano-8-methyl-4H-3,1-benzoxazin-4-one in an aromatic solvent system to a condensation reaction with methylamine followed by filtering, washing by water and drying.

In an embodiment, the present disclosure covers process for preparing a solid form of Cyantraniliprole which includes starting from an intermediate, 2-[3-Bromo-1-(3-chloro-2-pyridinyl)-1H-pyrazol-5-yl]-6-cyano-8-methyl-4H-3,1-benzoxazin-4-one, and converting it to a Cyantraniliprole solid state form, by a process, for example, including subjecting a solution of 2-[3-Bromo-1-(3-chloro-2-pyridinyl)-1H-pyrazol-5-yl]-6-cyano-8-methyl-4H-3,1-benzoxazin-4-one in an ether solvent system to a condensation reaction with methylamine followed by evaporation, drying under vacuum to obtain a solid. The said solid is further dissolved in an alcohol solvent, followed by cooling, precipitating, filtering and drying under vacuum to obtain a Cyantraniliprole solid state form, wherein the alcohol solvent may be selected from a group comprising Ethanol, Methanol, 1-Propanol and n-Butanol.

In further embodiment, the present disclosure covers process for preparing a solid form of Cyantraniliprole which includes starting from an intermediate, 5-Bromo-2-(3-chloro-2-pyridyl)-2H-pyrazole-3-carbonyl chloride, and converting it to a Cyantraniliprole solid state form, by a process, for example, including subjecting a solution of 5-Bromo-2-(3-chloro-2-pyridyl)-2H-pyrazole-3-carbonyl chloride in an aromatic solvent system to a condensation reaction with 2-Amino-5-cyano-N,3-dimethylbenzamide, followed by filtering, washing by an aromatic solvent and drying.

In another embodiment, the present disclosure encompasses the above described solid state forms of Cyantraniliprole for use in the preparation of agrochemical compositions and/or formulations, optionally for agronomic and non-agronomic uses, and a method of their use for controlling invertebrate pests in both agronomic and non-agronomic environments.

In another embodiment, the present disclosure encompasses the use of the described solid state form of Cyantraniliprole for the preparation of agrochemical compositions and/or formulations.

The present disclosure further provides agrochemical compositions including one or more solid state forms of Cyantraniliprole according to the present disclosure.

In yet another embodiment, the present disclosure encompasses agrochemical formulations including one or more of the described solid state forms of Cyantraniliprole and at least one additional component selected from the group consisting of a surfactant, a solid diluent and liquid diluent and optionally an effective amount of at least one additional biologically active compound or agent.

In an embodiment, the present disclosure encompasses processes to prepare said agrochemical formulations of Cyantraniliprole including one or more of the described solid state forms and at least one additional component selected from the group consisting of a surfactant, a solid diluent and liquid diluent and optionally an effective amount of at least one additional biologically active compound or agent.

In one embodiment, the solid state forms defined herein as well as the agrochemical compositions or formulations of the solid state forms of Cyantraniliprole can be used as systemic diamide insecticide for controlling invertebrate pests in both agronomic and non-agronomic environments.

In further embodiment, the present disclosure also encompasses methods for controlling invertebrate pests comprising applying solid state forms of Cyantraniliprole by activating muscle ryanodine receptors, causing contraction and paralysis of some pests.

In yet another embodiment, the present disclosure also covers uses of the solid state forms of Cyantraniliprole of the present disclosure, or at least one of the above agrochemical compositions or formulations for controlling invertebrate pests in both agronomic and non-agronomic environments.

Having described the disclosure with reference to certain preferred embodiments, other embodiments will become apparent to one skilled in the art from consideration of the specification. The disclosure is further illustrated by reference to the following examples describing in detail the preparation of the solid forms and methods of use of the disclosure. It will be apparent to those skilled in the art that many modifications, both to materials and methods, may be practiced without departing from the scope of the disclosure.

### Analytical Methods

### X-ray powder diffraction method:

Powder X-ray Diffraction was performed on an X-Ray powder diffractometer PanAlytical X'pert Pro; CuK_{α}, radiation, (λ = 1.54187 Å-); zero background sample holders. Measurement parameters:

| | |
|---|---|
| Scan range: | 4 - 40 degrees 2-theta |
| Scan speed | 3°/ min (for Form C, Form D); and 1.2°/ min (for Form F) |
| Sample holder: | Zero background silicon plate |

Prior to analysis, the samples were gently ground using a mortar and pestle to obtain a fine powder. Optionally, silicon powder can be added in a suitable amount as internal standard in order to calibrate the positions of the diffractions. The ground sample was adjusted into a cavity of the sample holder and the surface of the sample was smoothed using a cover.

### Examples

Solid forms of Cyantraniliprole were prepared according to the following procedures:

### Example 1: General procedure for the synthesis of the intermediate, 2-[3-Bromo-1-(3-chloro-2-pyridinyl)-1H-pyrazol-5-yl]-6-cyano-8-methyl-4H-3,1-benzoxazin-4-one:

In a three-necked flask equipped with a thermometer, a dropping funnel, and a reflux condenser, 3-bromo-1-(3-chloro-2-pyridinyl)-1H-pyrazole-5-carboxylic acid (3.4 g, 11.4 mmol) was suspended in ethyl acetate (25 ml), and the mixture was cooled in ice bath maintaining at a temperature below 25°C. Pyridine (5.2 g, 64.8 mmol, 5.7 eq.) was added dropwise while controlling that the temperature will stay below 25°C. Afterwards Methanesulfonyl chloride (neat 1.7 g, 14.8 mmol, 1.3 eq.) was added dropwise keeping temperature below 25⁰C. After full addition, the mixture was stirred for 10 min, followed by addition of 2-Amino-5-cyano-3-methylbenzoic acid (2.0 g, 11.4 mmol) dropwise maintaining the temperature below 25°C. After full addition, the mixture was stirred for 15 min. Second portion of Methanesulfonyl chloride (neat 1.7 g, 14.8 mmol, 1.3 eq.) was added dropwise keeping temperature below 25°C. After full addition, the mixture was heated to 70⁰C for 2h. The mixture was cooled to 0°C for 2 h in ice bath. The formed solid was filtered under vacuum and dried on the Büchner funnel for 30 min. Next the solid was washed by reslurry in water (50 ml) for 30 min at ambient temperature. Then the suspension was filtered under vacuum at ambient temperature. The solid was dried on the Büchner funnel for 30 min. The reslurry was repeated once more and the solid was washed by reslurry in water (50 ml) for 30 min at ambient temperature. Then the suspension was filtered under vacuum at ambient temperature. The solid was dried on the Büchner funnel for 30 min. followed by drying in a vacuum oven at 70⁰C for overnight. A yellow powder of 2-[3-Bromo-1-(3-chloro-2-pyridinyl)-1H-pyrazol-5-yl]-6-cyano-8-methyl-4H-3,1-benzoxazin-4-one was obtained in 70% yield (3.5 g).

Solid crystalline Forms of Cyantraniliprole were prepared according to following preparatory examples:

### Example 2: Preparation of Cyantraniliprole Form C

To a two-neck flask equipped with reflux condenser were added 2-[3-Bromo-1-(3-chloro-2-pyridinyl)-1H-pyrazol-5-yl]-6-cyano-8-methyl-4H-3,1-benzoxazin-4-one (1g, 2.259 mmol) and mono-chlorobenzene (4.44 g, 4 ml). The suspension was heated to 60°C. A solution of 40% MeNH₂ in H₂O (0.263 g, 0.4 ml, 1.5 eq., 3.388 mmol) was added dropwise under temperature control. At the addition of MeNH₂ the reaction mixture was turned into slurry. Additional amount of solvent was added and the solid was dissolved. Upon cooling solid was formed. To the formed solid in the flask was added mono-chlorobenzene (11.1 g, 10ml). The reaction was stirred at 60°C for 2h. At the end of reaction, the mixture was cooled to 0°C by ice bath and stirred for 2h at 0°C. Then the solid was filtered under vacuum. Next the solid was washed by re-slurrying in water (50 ml) at ambient temperature for 30 min. The solid was filtered under vacuum and dried in a vacuum oven at 70°C for overnight. A light-yellow powder was obtained in 85% yield (0.912 g). Solid was then gently ground with spatula and analysed by XRD.

### Example 3: Preparation of Cyantraniliprole Form D

In a flask,2-[3-Bromo-1-(3-chloro-2-pyridinyl)-1H-pyrazol-5-yl]-6-cyano-8-methyl-4H-3,1-benzoxazin-4-one (0.959 g, 2.027 mmol) was suspended in THF (42.67 g, 48 ml) at ambient temperature. Over the course of 15 minutes a solution of MeNH₂ (2.0 M solution in THF, 4.8ml, 9.6 mmol) was added dropwise and the reaction mixture was stirred for 40 minutes. Then the THF was evaporated under reduced pressure until dryness. During the evaporation the solid formed. The solid was dried in a vacuum oven at 65°C for overnight. An off-white powder was obtained in 97% yield (0.932 g). In Erlenmeyer flask, the off-white powder (0.5 g,1.057mmol) was dissolved by a minimum amount of hot 1-propanol, which was added in portions until all the powder was dissolved. Then the solution was cooled to 0°C by ice bath up to the appearance of precipitate. The solid was filtered under vacuum and dried in a vacuum oven at 65°C for overnight. White powder was obtained. Solid was then taken in a spatula and analysed by XRD.

### Example 4: Preparation of Cyantraniliprole Form F

In a three-necked flask equipped with a thermometer, a dropping funnel, and a reflux condenser, 3-bromo-1-(3-chloro-2-pyridinyl)-1H-pyrazole-5-carboxylic acid (1.0 g, 3.3 mmol, 1 eq.) was suspended in toluene (6 ml), and the mixture was heated to 80°C. SOCl₂ (0.47 g, 4.0 mmol, 1.2 eq.) was added dropwise, and the resulting mixture was heated for 1.5 h, at which time the mixture was clear. Toluene (6 ml) was added, the reaction mixture was cooled to RT, and ca. 6 ml of the solvent was evaporated under reduced pressure to obtain crude 5-Bromo-2-(3-chloro-2-pyridyl)-2H-pyrazole-3-carbonyl chloride, which is used for further step.

In a three-necked flask, equipped with a thermometer, a dropping funnel, and a vacuum distillation setup, 2-Amino-5-cyano-N,3-dimethylbenzamide (0.66 g, 3.5 mmol, 1.05 eq.) was suspended in toluene (3 ml), the pressure in the system was adjusted to 550 mbar, and the mixture was heated so that the solvent distilled slowly (reflux, bath temperature: 115-120⁰C, internal temperature: 85°C). The solution of 5-Bromo-2-(3-chloro-2-pyridyl)-2H-pyrazole-3-carbonyl chloride in toluene was added dropwise over 30 min, and the mixture was left to distil, with toluene being added as necessary, so that all solids remained suspended in the solvent. After 2h, TLC (2% MeOH/DCM) showed full conversion. The reaction mixture was cooled to RT, filtered, washed with toluene, and the resulting white solid was air-dried, giving 1.45 g of the wet product (93%). An aliquot was dried at 2 mbar and 40⁰C until constant dried mass with 93% yield. Both wet and dry solid were then taken in a spatula and analysed by XRD. X-Ray powder diffraction showed that the sample, both wet and dry, is crystallised in form F.

### Example 5: Preparation of Cyantraniliprole Form D from Form F

Procedure 1: 1.0 g of dry Form F of Cyantraniliprole was stirred in 10 ml of 1-propanol for 18h, followed by filtering and drying at 90°C under vacuum to obtain Cyantraniliprole Form D, analysed by XRD.

Procedure 2: 1.0 g of dry Form F of Cyantraniliprole was stirred in 3.2 g of 1-propanol for 1h at 85-90°C with air bubbled through dip-pipe, followed by cooling to 0°C, filtering and drying at 90°C under vacuum to obtain Cyantraniliprole Form D, analysed by XRD.

Procedure 3: 1.0 g of dry Form F of Cyantraniliprole was stirred in 3.2 g of 1-propanol for 1h at 85-90°C with air bubbled through dip-pipe, followed by cooling to 0°C, filtering, washing with cold 1-propanol and drying at 90°C under vacuum to obtain Cyantraniliprole Form D, analysed by XRD.

Procedure 4: 1.0 g of dry Form F of Cyantraniliprole was stirred in 3.2 g of 1-propanol for 1h at 85-90°C with air bubbled through dip-pipe, followed by cooling to 0°C, filtering, washing with cold 1-propanol and drying at 120°C under vacuum to obtain Cyantraniliprole Form D, analysed by XRD.
The present invention is further defined by the following items:
1. A Crystalline Form C of Cyantraniliprole, which is characterized by data selected from one or more of the following:
   i. an XRPD pattern having peaks at 7.3, 8.2, 8.9, 14.7 and 20.3 degrees 2- theta ± 0.2 degrees 2-theta;
   ii. an XRPD pattern as depicted in Figure 1.
2. The crystalline form of Cyantraniliprole according to item 1, which is characterized by an XRPD pattern having peaks at 7.3, 8.2, 8.9, 14.7 and 20.3 degrees 2-theta ± 0.2 degrees 2- theta, and also having one, two or three additional peaks selected from 16.3, 18.7, 24.5 and 25.4 degrees 2-theta ± 0.2 degrees 2-theta.
3. A Crystalline Form D of Cyantraniliprole, which is characterized by data selected from one or more of the following:
   i. an XRPD pattern having peaks at 6.6, 11.4, 15.7 and 22.4 degrees 2- theta ± 0.2 degrees 2-theta;
   ii. an XRPD pattern as depicted in Figure 2.
4. The crystalline form of Cyantraniliprole according to item 3, which is characterized by an XRPD pattern having peaks at 6.6, 11.4, 15.7 and 22.4 degrees 2-theta ± 0.2 degrees 2-theta, and also having one, two or three additional peaks selected from 14.7, 19.6, 21.4 and 25.7 degrees 2-theta ± 0.2 degrees 2-theta.
5. A Crystalline Form F of Cyantraniliprole, which is characterized by data selected from one or more of the following:
   i. an XRPD pattern having peaks at 9.6, 11.3, 18.6, 21.5 and 25.9 degrees 2- theta ± 0.2 degrees 2-theta;
   ii. an XRPD pattern as depicted in Figure 3.
6. The crystalline form of Cyantraniliprole according to item 5, which is characterized by an XRPD pattern having peaks at 9.6, 11.3, 18.6, 21.5 and 25.9 degrees 2-theta ± 0.2 degrees 2- theta, and also having one, two or three additional peaks selected from 15.1, 16.7, 19.7, 20.3 and 26.5 degrees 2-theta ± 0.2 degrees 2-theta.
7. An agrochemical composition comprising a crystalline form according to any one of items 1-6.
8. Use of a crystalline form according to any one of items 1-6 in the preparation of an agrochemical composition and/or formulation.
9. An agrochemical formulation comprising a crystalline form according to any one of items 1-6 or an agrochemical composition of item 7, and at least one agrochemically acceptable excipient.
10. A crystalline form according to any one of items 1-6, an agrochemical composition according to item 7, or an agrochemical formulation according to item 9, for the use as an insecticide in agronomic and non-agronomic environment.
11. A crystalline form according to any one of items 1-6, an agrochemical composition according to item 7, or an agrochemical formulation according to item 9, for use in controlling invertebrate pests in both agronomic and non-agronomic environments.
12. Use of a crystalline form according to any one of items 1-6, an agrochemical composition according to item 7, or an agrochemical formulation according to item 9, for the use as an insecticide in both agronomic and non-agronomic environments, preferably for the use in controlling invertebrate pests in both agronomic and non-agronomic environments.
13. Use of any of a crystalline form according to any one of items 1-6 for preparing any other crystalline form of Cyantraniliprole.
14. The use according to item 13, which is characterized by a use of Cyantraniliprole Form F for preparing Form D of Cyantraniliprole.
15. A process of preparation of the crystalline form of Cyantraniliprole according to item 1 or item 2, comprising subjecting a solution of 2-[3-Bromo-1-(3-chloro-2-pyridinyl)-1H-pyrazol-5-yl]-6-cyano-8-methyl-4H-3,1-benzoxazin-4-one in an aromatic solvent system to a condensation reaction with methylamine followed by filtering, washing by water and drying.
16. A process of preparation of the crystalline form of Cyantraniliprole according to item 3 or item 4, comprising subjecting a solution of 2-[3-Bromo-1-(3-chloro-2-pyridinyl)-1H-pyrazol-5-yl]-6-cyano-8-methyl-4H-3,1-benzoxazin-4-one in an ether solvent system to a condensation reaction with methylamine followed by evaporation, drying under vacuum to obtain a solid, wherein said solid is further dissolved in an alcohol solvent, followed by cooling, precipitating, filtering and drying under vacuum to obtain the said solid state form of Cyantraniliprole.
17. The process of preparation of the crystalline form of Cyantraniliprole according to item 16, wherein in the process the alcohol solvent may be selected from a group comprising Ethanol, Methanol, 1-Propanol and n-Butanol.
18. A process of preparation of the crystalline form of Cyantraniliprole according to item 5 or item 6, comprising subjecting a solution of 5-Bromo-2-(3-chloro-2-pyridyl)-2H-pyrazole-3-carbonyl chloride in an aromatic solvent system to a condensation reaction with 2-Amino-5-cyano-N,3-dimethylbenzamide, followed by filtering, washing by an aromatic solvent and drying.

## Claims

1. A Crystalline Form F of Cyantraniliprole, which is **characterized by** an XRPD pattern having peaks at 9.6, 11.3, 18.6, 21.5 and 25.9 degrees 2-theta ± 0.2 degrees 2-theta.

2. The crystalline form of Cyantraniliprole according to claim 1, which is **characterized by** an XRPD pattern having peaks at 9.6, 11.3, 18.6, 21.5 and 25.9 degrees 2-theta ± 0.2 degrees 2-theta, and also having one, two or three additional peaks selected from 15.1, 16.7, 19.7, 20.3 and 26.5 degrees 2-theta ± 0.2 degrees 2-theta.

3. An agrochemical composition comprising a crystalline form according to claim 1 or claim 2.

4. An agrochemical formulation comprising a crystalline form according to claim 1 or claim 2 or an agrochemical composition of claim 3, and at least one additional component selected from the group consisting of a surfactant, a solid diluent and liquid diluent and optionally an effective amount of at least one additional biologically active compound or agent.

5. A process of preparation of the crystalline form of Cyantraniliprole according to claim 1 or claim 2, comprising subjecting a solution of 5-Bromo-2-(3-chloro-2-pyridyl)-2H-pyrazole-3-carbonyl chloride in an aromatic solvent system to a condensation reaction with 2-Amino-5-cyano-N,3-dimethylbenzamide, followed by filtering, washing by an aromatic solvent and drying.
